(19) Europäisches Patentamt
European Patent Office
Office européen des brevets

(11) Publication number: **0 170 210 B1**

(12) **EUROPEAN PATENT SPECIFICATION**

(45) Date of publication of patent specification: **18.09.91**  (51) Int. Cl.⁵: **B01D 63/00**

(21) Application number: **85109348.4**

(22) Date of filing: **25.07.85**

(54) **Novel mat structure.**

(30) Priority: **01.08.84 US 636755**

(43) Date of publication of application:
**05.02.86 Bulletin 86/06**

(45) Publication of the grant of the patent:
**18.09.91 Bulletin 91/38**

(84) Designated Contracting States:
**BE CH DE FR GB IT LI NL**

(56) References cited:
**DE-A- 2 721 444        DE-A- 2 825 065**
**DE-A- 3 304 353        FR-A- 2 326 956**
**US-A- 2 484 003        US-A- 3 277 564**
**US-A- 3 690 465**

(73) Proprietor: **PPG INDUSTRIES, INC.**
**One PPG Place**
**Pittsburgh Pennsylvania 15272(US)**

(72) Inventor: **Smoot, Michael Alexander**
**1000 S Woodlawn Apt. 407**
**Wichita Kansas 67218(US)**
Inventor: **Barch, Herbert William**
**1306 Dallas Avenue**
**Natrona Heights Pennsylvania 15065(US)**
Inventor: **Das, Balbhadra**
**8005 Lingay Drive**
**Allison Park Pennsylvania 15101(US)**

(74) Representative: **Sternagel, Hans-Günther, Dr.**
**et al**
**Patentanwälte Dr. Michael Hann Dr. H.-G.**
**Sternagel Sander Aue 30**
**W-5060 Bergisch Gladbach 2(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

In many filtration processes today membranes are utilized to filter various components of fluid systems. For example, membranes are used to separate gas components from each other in gaseous streams containing multiple gases, to separate various dissolved components in liquid solutions from each other and to selectively permit certain ions in a solution to pass across a membrane while blocking others. Membranes are also utilized to a great extent to immobilize proteins, enzymes and cells. The enzymes, so immobilized are used as catalysts to increase reaction rates or to convert materials in solution from one form to another. Membranes are also utilized in various applications today to trap or immobilize living cells within a substrate forming the membrane.

In general, membranes of various types have been employed for these purposes. In the electrolysis field, for example, polymer sheet membranes which are selectively permeable to alkali metal ions are utilized. Porous glass beads have also been employed in many processes for the purpose of immobilizing enzymes for use in other chemical processes. Organic fibers have also been utilized in many applications, for example, the dialysis of blood. These organic fibers have been utilized both in the hollow and porous state where the material to be purified, in this case blood, is passed through a hollow organic fiber and is purified by enriching it in oxygen and depleting it of waste materials through the pores.

Inorganic materials are particularly interesting for membrane applications since they are, generally speaking, inert and depending on composition, alkali or acid resistant. These properties render such inorganic materials useful in purification systems that are acidic or alkaline. Further, their inertness renders such inorganic materials useful in cell, protein and enzyme immobilization since they are non-reactive to these substances and also to contaminants such as microorganisms that might be present in solutions being treated. Inorganic substances further can be readily cleaned without suffering severe damage during cleaning and sterilization, whereas many organic substances cannot be cleaned using normal cleaning materials such as calcium hypochlorite solutions. Interest in inorganic substrates in the form of hollow glasses which are porous is demonstrated by an article in "The Journal of Material Science" (11), 1976 at pages 1187-1199 by P. W. McMillan and C. E. Matthews. The recent U S -A- 4,042,359 also shows a device made of porous glass tubes. These devices use individual tubes in what appears to be limited capacity reactors since the tubes are separated from each other with each tube restrained at each end.

It is the object of the invention to provide a glass fiber composite mat having a structure that can be effectively utilized in reverse osmosis, microfiltration, ultrafiltration, enzyme, protein and cell immobilization and other like processes in a commercial reactor to provide a large number of porous glass fibers for use in the process being conducted.

The object is attained by a glass fiber containing composite mat characterized by having a plurality of glass fibers affixed to one of the major surfaces of a fluid permeable sheet having two major surfaces and four edge surfaces, said fibers being aligned generally parallel to each other with their ends facing two opposite edges of the major surface of said sheet and at least two strips of an adhesive parallel to and spaced from each other and inboard of the ends of said fibers, said adhesive strips affixing the fibers to said sheet and to each other to thereby form an integral, composite mat wherein said fibers have pores therein or are hollow nonporous fibers or hollow fibers throughout their lengths also having pores.

This object is also attained by a cartridge for the use in fluid separation systems having an upper casing member forming the top of the cartridge casing and a lower casing member in which the strands of hollow fibers and the permeable sheets of a composite mat according to the invention are cast and ends of the fibers in the upper casing member are sealed by casting and wherein the adhesive strips are positioned just below the upper casing member and just above the lower casing member as barriers and the permeable sheet is wraped around a distributor tube centrally disposed to the strands and terminating and embedded in the upper and lower casing members wherein the end of the distributor tube embedded into the lower casing member is sealed by casting and followed by a layer of hollow strands and then alternative layers of sheet and the hollow strands as the mat is wrapped in successive wraps around the distributor tube.

This cartridge is used in a fluid separation device having a tubular casing which has a fluid inlet and a fluid outlet line and a cover member associated therewith and sealed with the side walls of the casing wherein the cartridge is positioned within the casing and the distributor tube is connected with the fluid inlet and the lower casing member of the cartridge is sealed to the side walls of the separation.

Applicants, by virtue of the instant invention, have supplied that need by providing the art with novel and useful mats utilizing as major components thereof glass fibers in the form of fibers, glass fiber strands, which comprise groups of glass fibers, and glass fiber rovings which comprise groups of glass fiber strands. The glass fibers used to produce the mats whether they are used as fibers, strands or rovings, are porous glass fibers, hollow glass fibers or hollow glass fibers, which are also porous.

The mats made from these fibers may be utilized in forming cartridges for use in filtration apparatuses for gas and/or liquid separations, for reverse osmosis and ultrafiltration systems, as a carrier for cell cultures in reactors requiring large flat surface areas for cell growth, as elements in systems designed for the immobilization of proteins and enzymes, as blood dialysis membranes and other such systems. The number of fibers used to prepare the mats provide hundreds of thousands to millions of individual glass fibers in a form readily adaptable for use in various filtration and immobilization reactors.

Thus, the mats of the instant invention provide in convenient form, large quantities of glass fibers, which by chemical composition, can be tailored to various media to which they may be exposed to render them resistant to reactant attack in a given process. They may also be varied in the pore sizes used, where the fibers are porous, to provide specific mats for use in ultrafiltration; reverse osmosis and the like. Fiber size, i.e., diameter, can be varied to provide, in the case of hollow fibers, an internal lumen of a specified or given size with or without the fibers also being porous, thereby rendering mats made from such fibers useful in dialysis systems as well as cell culture reactors.

In accordance with one embodiment of the invention, a novel mat composite is provided in which a plurality of porous glass fibers in the form of fibers, strands or rovings are carried on a generally flat, fluid permeable sheet. The fibers, strands or rovings are generally aligned on the surface of the permeable sheet parallel to each other. Strips or ribbons of an adhesive are provided between the ends of the fibers, strands or rovings, and aligned on the surface of the fluid permeable sheet to attach or affix the fibers to the surface of the sheet and to each other to thereby form an integral composite mat containing porous glass fiber in the form of fibers, strands or rovings.

In another embodiment of the invention, a second fluid permeable sheet is placed over the fibers, strands or rovings and is of a length and width sufficient to cover the first several rows of fibers, strands or rovings attached to the first permeable membrane.

The various embodiments of the inventions will be apparent to one having ordinary skill in the art from consideration of the ensuing description and claims.

BRIEF DESCRIPTION OF THE DRAWINGS

For a more complete understanding of the instant invention, reference is made to the accompanying drawings in which:
Fig. 1 is a plan view of the mat structure of the instant invention;
Fig. 2 is a cross-section of the mat of Fig. 1 taken along lines II-II;
Fig. 3 is a diagrammatic illustration of the mat of Fig. 1 shown used in a cartridge; and
Fig. 4 is a diagrammatic illustration of the cartridge of Fig. 3 in a filtration chamber.

DETAILED DESCRIPTION OF THE DRAWINGS

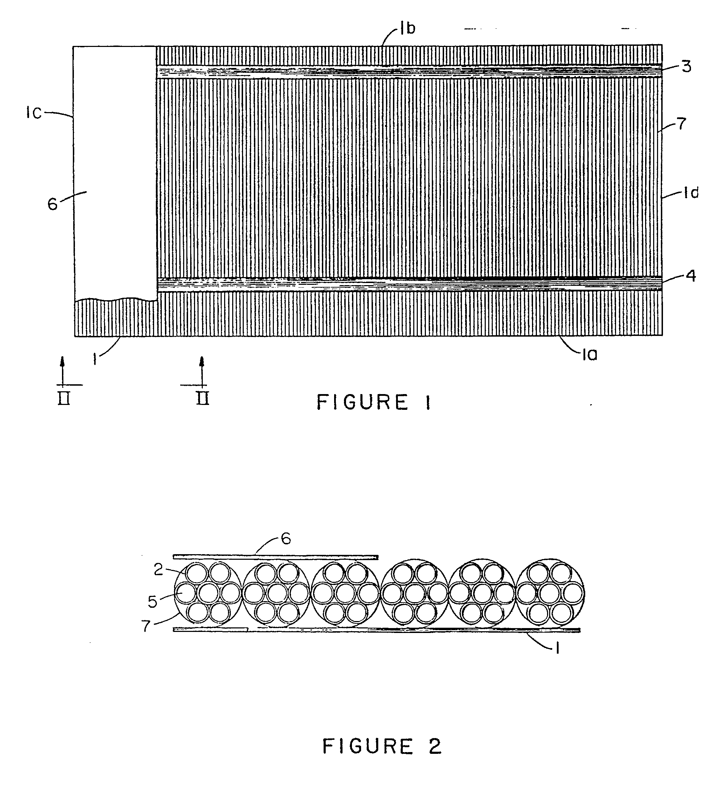

Turning now to the drawings and to Fig. 1 and Fig. 2 in particular, the invention will be described as it applies to an embodiment in which strands of glass fibers are used to form the mat of the invention. It will be understood that the fibers used in the strands are made of glass. While in this embodiment, the fibers are hollow, they can be porous only or porous and hollow or a combination of all these forms or two of the three forms and still fall within the scope of the invention. Similarly, while strands of hollow, glass fibers are used in the drawing, the mat shown can be made of individual fibers or rovings as well as the strands shown or a combination of two or more of the group of fibers, strands and rovings.

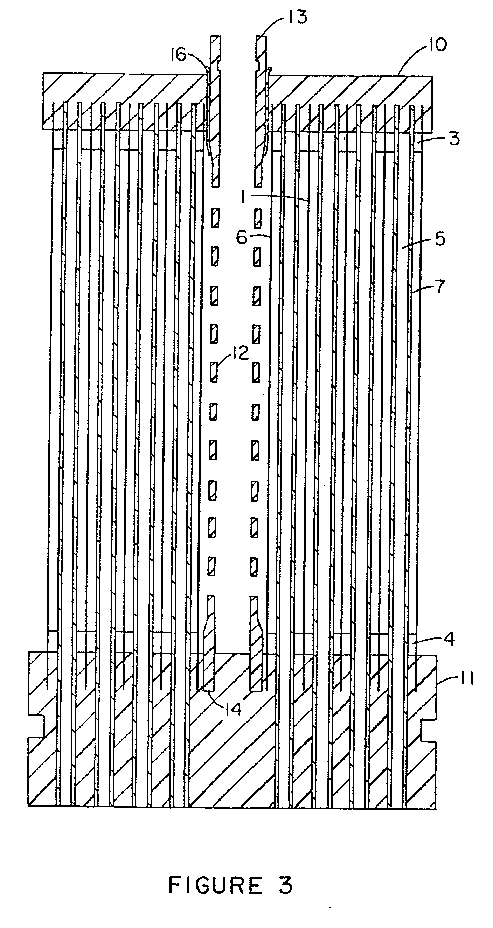

In Fig. 1, there is shown a flat, fluid permeable sheet 1 on which are positioned a plurality of hollow glass fiber strands 7 which are fixed to the surface of the permeable sheet 1 and which are in parallel alignment with each other and edges 1c and 1d of the permeable sheet 1. Extending longitudinally along the permeable sheet 1 and parallel to edges 1a and 1b thereof are two adhesive strips or ribbons 3 and 4. Strips or ribbons 3 and 4 are formed of an adhesive material which is of sufficient depth and width to cohesively bond the strands 7 to one another to keep them in parallel alignment. The strips 3 and 4 also prevent resin wicking during the casting of the mats into cartridges such as shown in Fig. 3. The ribbons 3 and 4 also bond the strands 7 to the permeable sheet 1.

Fig. 2 is an enlarged view of a cross-section of Fig. 1 taken along line II-II showing the hollow fibers 2 which make up the strands 7. The fibers 2 as shown have a lumen 5 which runs the length of the fibers 2. As illustrated in the drawing, since strands 7 are employed on the surface of permeable sheet 1, a plurality of hollow fibers 2 (seven in the illustration) are arranged in parallel in each of the strands 7. Also shown in Fig. 2 is the inclusion of a second fluid permeable sheet 6, which is of a short width and overlays the first several rows of strands 7 affixed to the permeable membrane 1. The purpose of the permeable sheet 6 is to provide a permeable membrane at one end of the mat so that it can be utilized in a cartridge for use in a filtration or dialysis system through which fluid is introduced through a distributor tube. The sheet 6 protects the first layer of strands from damage by dissipating fluid flow forces entering the mat when it is wrapped around a fluid distributor in a cartridge for example. If desired, the sheet 6 can encompass the entire strand layer.

Fluid permeable sheet as used herein is intended to mean any form of structure such as woven or nonwoven mats, cloth, paper and the like, which are pervious to fluid flow through their surfaces, whether the fluid is liquid or gaseous, and which are resistant to attack by the fluid to which they are subjected. Utilization of materials such as fiber glass filament mats, and papers, polyester fiber mats, woven or knitted cloth made of synthetic fibers, glass, cotton and the like can be used. The important consideration for the selection of the material used as the fluid permeable sheet is that it be constructed so that it will support the fibers, strands and rovings to which it is attached and permit free fluid flow through it. The major purpose of the sheet is to protect the fibers, strands and rovings from damage caused by fluid flow forces and by abrasion with each other and with surfaces around which the mats of the invention may be wrapped or pressed against in service.

Materials that can be utilized as the ribbons 3 and 4 may consist of hot melt thermoplastic resins or thermoset resins. Some examples of hot melt thermoplastics would be homopolymers or copolymers of polyvinyl acetate, acrylates, acrylonitriles, polysulfones, polyamides and the like. Examples of some thermosets that may be employed are anhydride or amine curable epoxy resins, peroxide curable polyesters, polyimides, and various copolymers of these polymers. These polymers may be dissolved in some solvent and may be contained in a tube with the catalyst. This catalyst can be activated in the presence of heat, oxygen, water or various environments of this nature.

The hollow fibers utilized in the mat of the instant invention are glass fibers which have been prepared in such a manner that they are provided with a lumen from one end of the fiber to the other end so that fluid can flow from one end of the fiber to the other end of the fiber unimpeded. A convenient method of preparing hollow fibers is described in assignee's issued U S -A-3,268,313. Particular glass fibers that can be used are described in U S -A- 3,510,393.

The glass composition forming the fibers, as far as the instant invention is concerned, is not of paramount importance and any glass composition suitable for use in making glass fibers which can be drawn into hollow structures as described in the aforesaid U.S. patents is suitable. Typical glasses which may be employed for this purpose are "E" or "621" glasses and/or other borosilicate glasses containing from 8 to 28 percent $B_2O_3$ or more on a weight basis of the glass composition. Glasses of these types are described in U S -A-2,106,744; 2,334,961; 2,571,074; 3,650,721. Glasses having low $B_2O_3$ such as described in U S -A- 4,166,747 as well as glasses not containing either fluorine or boron such as described in U S-A-3,847,626.

In those instances, where porous fibers are employed, the porosity is provided to the glass fibers by employing any of many well known techniques to the skilled art. Thus, in treating borosilicate glass, the glass is typically heat treated for a given period of time after which it is treated with a mineral acid to leach out the borosilicate rich phase to provide pores of specific diameter. This system is described in Assignees' U S -A- 3,630,700 in connection with glass particles, but the systems also apply to treatments involving glass fibers. Assignees' U S -A- 3,650,721 shows a system of treating fibers of a boron containing glass which renders them porous using a similar heat treatment followed by an acid leach. Similar treatments to provide porosity to glass fibers are also described in U S -A- 4,042,359. In utilizing the principles described in these patents, glass fibers which are solid or hollow can be treated to provide porosity to the fibers. In the case of hollow fibers, where it is desired, the leaching is normally conducted for a sufficient time to provide pores that communicate with the lumen of the hollow fibers. The treatment of fibers to render them porous can be conducted while the fibers are in fiber, strand or roving form or can be conducted while the fibers, strands and rovings are in mat form. It is preferred by Applicants to render fibers porous after they are in mat form and most preferably after they are in a cartridge form such as shown in Fig. 3.

Turning to Fig. 3, an assembly of the mat of Fig. 1 is shown in which the hollow strands 7 are aligned vertically in a catridge that may be used in a fluid separation system. The cartridge involves an upper casing member 10 in which the strands 7 and the fluid permeable sheets 1 and 6 are cast. The adhesive barrier 3 is located just below the casing member 10. Similarly, the hollow strands 7 are also shown cast in a lower casing member 11 and the adhesive barrier 4 is positioned just above the casing member 11. This barrier 4 serves to prevent wicking of resin into the fibers during the casting of member 11. The lumen 5 shown is for illustrative purposes, it being understood that this represents the lumen of each fiber contained in strands 7. The fluid permeable sheet 6 is wrapped around a distributor tube, generally indicated as 12, that runs in a generally centrally disposed relationship to the hollow strands 7 contained in the cartridge and terminates in the casting 10 forming the top of the cartridge casing. The other end 14 of the distributor tube 12 is embedded in the bottom casing 11. As can be readily seen from the drawing, the first membrane 6 is wrapped completely around the distributor tube 12 and is followed by a layer of hollow strands 7 and then alternate layers of the sheet 1 and the hollow strands 7 as the mat is wrapped in successive wraps around distributor tube 12. A thin plastic sheet 16 is provided around collar 13 at the top of the cartridge.

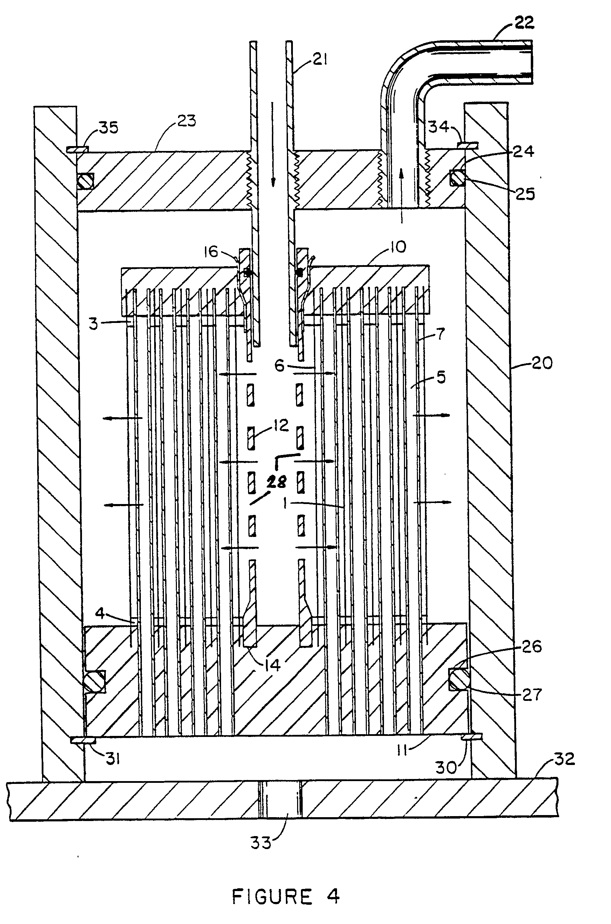

In an operation in which a fluid separation is being conducted, a fluid separation device such as shown in Fig. 4 is utilized. The fluid separation device of Fig. 4 involves a tubular casing 20 which has a fluid inlet 21, a fluid outlet line 22 and a cover member 23 associated therewith. Cover member 23 is sealed with respect to the sidewalls of the casing 20 utilizing grooves 24 and appropriate gasketing 0 rings 25 at the top. On the bottom, the cartridge itself has a groove 26 associated therewith in which is placed an 0 ring 27 to seal the bottom casing of the cartridge to the walls separation unit. In the embodiment shown, the fibers forming the strands 7 are porous as well as being hollow and fluid is fed through inlet 21 into the distributor tube 12 and passes, as shown by the arrows, through the openings 28 in the distributor tube 12 and through the walls of the porous hollow fibers contained in strands 7. The material passing through walls of the fibers contained in strands 7, pass through the lumen 5 of the hollow fibers contained in strands 7 and exits at the end of the resin member 11. The collar 14 of distributor tube 12 is embedded in the member 11 so that fluid entering line 21 must exit through the openings 28 in order to be removed from the system. Fluid that does not pass down through the lumen 5 of the hollow fibers in strands 7 passes to the outside of the cartridge containing the strands 7 and passes between the wall of the casing 20 and the outside of the strands 7 forming the cartridge and upwardly into the feed return duct 22 and out of the system.

In preparing porous glass fibers for use in the mats of the instant invention, recourse to several methods may be had. If the glass fibers, strands or rovings in the mat are made of an "E" or "621" glass composition, the mat may be used in a cartridge such as shown in Fig. 3. In this instance, the cartridge may be placed in a unit such as shown in Fig. 4. The inlet tube 21 is capped and the exit 33 is plugged. The vessel 20 is filled to the cap 10 area with 3 N HCl and maintained in the vessel for 0.5 to 5 hours at 40 to 95° C. The vessel 20 is then emptied, flushed with distilled water and is ready for use. It is an important consideration that the plastic film 16 be present around the collar of distributor tube 12 during leaching the glass strands 7 since they tend to shrink during treatment. This film, which may be thin Mylar® or other plastic materials such as polyethylene, polypropylene, polyethylene terephthalate, Teflon® and the like, permits cap 10 to move downwardly as the strands 7 shrink to thereby minimize any fracturing of the strands.

In another method using an "E" or "621" glass composition, the mat is used in cartridge form such as Fig. 3 and placed in a vessel such as Fig. 4. In this method, the mat is leached by passing the leaching acid, typically 3 N HCl, into inlet tube 21 and removing it through outlet 22. In the alternative, the leaching acid can be passed into the vessel through line 22 and removed through line 21. This circulating acid is typically fed for 0.5 to 5 hours at temperatures of 40 to 95° C.

In instances where high boron containing glasses are to be treated, the mats are heat treated to phase separate the glass. This is done by subjecting the mat to temperatures of 200 to 750° C in an oven for a period of time sufficient to form silica rich and borosilicate rich phases in the glass, typically from 5 minutes to 24 hours. Since the adhesive may not withstand the oven temperatures, it may be necessary to reapply the adhesive ribbons 3 and 4 before leaching the phase separated mat. Preferably, after the mat is phase separated, it is formed into a cartridge such as Fig. 3 and placed in a reactor similar to Fig. 4. Once the mat is in place in a vessel such as Fig. 4, it may then be leached by the methods above described for the "E" and "621" glasses.

In the alternative, the fiber strands and rovings can be heat treated before forming them into a mat and subsequently assembled in mat form.

The acids used are typically inorganic mineral acids such as HCl, $H_2SO_4$ and $H_2NO_3$ at normalities of 1 to 6. Strong organic acids such as citric acid may also be used but mineral acids are preferred.

If desired, the mats of "E" or "621" glasses as well as the phase separated borosilicate glasses may be leached by exposing them to the acid treatment in the mat form rather than in cartridge form so long as they are treated for the times and temperatures indicated for the cartridge treatments.

The following is an example of the method used to construct a mat similar to Fig. 1 of hollow glass fibers in roving form.

## EXAMPLE

A mat similar to that shown in Fig. 1 was prepared as follows:

Hollow fiber glass strands prepared by the process of U S -A- 3,268,313 and contained in a roving package are wound onto a drum. The drum used was manufactured by C. A, Litzler Co. of Cleveland, Ohio. It is 122 cm (48 inches) wide and has a 122 cm (48 inch) diameter. Prior to winding, a clear polypropylene sheet is taped to the drum surface. This prevents the adhesive, which is applied later, from sticking to the winder. A 254 μm (10 mil), fluid permeable, polyester surface mat (Dupont's Reemay®) mat is taped to the drum winder, over the polypropylene sheet. The porous polyester mat forms the support backing for the yet to be wound glass roving. A roving containing E-glass fibers with 2% epoxy sizing on the fibers is used to supply fibers for a mat. The roving consists of 40 strands, each strand containing 102 individual hollow fibers whose dimensions are approximately 12 μms O.D. and 6 μm I.D.

The roving is wound onto the drum and results in 14 rovings per 25.4 mm (inch), or 560 strands per 25.4 mm (inch) or 57,120 fibers per 25.4 mm (inch). The roving is continuously wound onto the drum with the rovings generally parallel to each other until the drum is covered. The mat resulting is measured and marked at 330 mm (13 inch) sections on the drum. Each mat section will come from the 33 x 111.8 (13 cm inch x 44 inch) pieces [50:8 mm (2 inches)are lost at each end of the drum]. Prior to cutting the mat and removing it from the drum, the adhesive strips 3 and 4 are applied. In this instance, a contact cement manufactured by Franklin Chemical of Columbus, Ohio is used.

Two 12,7 mm (1/2 inch) adhesive lines are applied to each mat section. One line is 25.4 mm (one inch) from the end, the other is 63.5 mm (2 1/2 inches) from the other end. The adhesive lines run perpendicular to the fiber direction and serve to both bond the fibers together and to the polyester fluid permeable sheet as well as serving later to prevent resin wicking. Once the adhesive has dried, the blanket is cut at one of the mat locations. This permits removal of the blanket from the drum winder. The blanket is laid onto a cutting table, and a 33 x 55.9 cm (13 inch x 22) inch hollow fiber mat is cut. Using this procedure, a mat is provided which, when used in a 50.8 mm (2") diameter module or cartridge such as shown in Fig. 3 has available for use in that cartridge, 370,480 hollow fibers.

As will be readily appreciated, the mats of the instant invention provide a source of hollow fibers or hollow porous fibers or porous fibers in a mat form which, can be made to accommodate in a small area extremely large quantities of these fibers for use in commercial filtration and immobilization reactors. In instances where cartridges of layer dimensions are employed, it will be appreciated that vast amounts of fibers will be present. For example, in a 101.6 mm (4") diameter module, it would typically require the use of mats that would provide typically 2,227,000 fibers.

## Claims

1. A glass fiber containing composite mat characterized by having a plurality of glass fibers (2) affixed to one of the major surfaces of a fluid permeable sheet (1) having two major surfaces and four edge surfaces (1a, 1b, 1c, 1d), said fibers (2) being aligned generally parallel to each other with their ends facing two opposite edges of the major surface of said sheet (1) and at least two strips (3, 4) of an adhesive parallel to and spaced from each other and inboard of the ends of said fibers, said adhesive strips (3, 4) affixing the fibers (2) to said sheet (1) and to each other to thereby form an integral, composite mat wherein said fibers (2) have pores therein or are hollow nonporous fibers or are hollow fibers throughout their length also having pores.

2. The composite mat of claim 1, wherein the fibers (2) are in the form of strands (7).

3. The composite mat of claim 1, wherein the fibers (2) are in the form of rovings.

4. The composite mat of claims 1 to 3, wherein a second permeable sheet (6) is placed over several rows of said fibers (2) at one end of said mat covering the whole length of said fibers (2) between the two adhesive strips (3, 4).

5. Cartridge for the use in fluid separation systems having an upper casing member (10) forming the top of the cartridge casing and a lower casing member (11) in which the strands (7) of hollow fibers (2) and the permeable sheets (1, 6) of a composite mat according to claims 1-2 and 4 are cast and ends of the fibers in the upper casing member (10) are sealed by casting and wherein the adhesive strips (3, 4) are positioned just below the upper casing member (10) and just above the lower casing member (11) as barriers and the permeable sheet (6) is wraped around a distributor tube (12) centrally disposed to the strands (7) and terminating and embedded in the upper and lower casing members (10, 11) wherein the end of the distributor tube (12) embedded into the lower casing member (11) is sealed by casting and followed by a layer of hollow strands (7) and then alternative layers of sheet (1) and the hollow strands (7) as the mat is wrapped in successive wraps around the distributor tube (12).

6. Cartridge of claim 5,
   wherein the fibers strands (7) of the mat are hollow and having pores.

7. Use of the cartridge according to claims 5 or 6 in a fluid separation device having a tubular casing (20) which has a fluid inlet (21) and a fluid outlet line (22) and a cover member (23) associated therewith and sealed with the side walls of the casing (20) wherein the cartridge is positioned within the casing (20) and the distributor tube (12) is connected with the fluid inlet (21) and the lower casing member (11) of the catridge is sealed to the side walls (20) of the separation device.

## Revendications

1. Mat composite, qui contient des fibres de verre, caractérise en ce qu'il possède une multiplicité de fibres de verre (2) fixées à l'une des surfaces principales d'une feuille (1) perméable aux fluides, comportant deux surfaces principales et quatre surfaces formant bord ou bordure ou lisière (1a, 1b, 1c, 1d), les fibres (2) étant alignées, dans leur ensemble, parallèlement les unes aux autres, leurs extrémités faisant face à deux bords opposés de la surface principale de ladite feuille (1) et au moins deux bandes (3, 4) d'un adhésif, parallèles et espacées l'une de l'autre et à l'intérieur des extrémités desdites fibres, les bandes adhésives (3, 4) fixant les fibres (2) à la feuille précitée (1) et les unes aux autres, de manière à ainsi former un mat composite intégral ou venu de matière dans lequel les fibres précitées (2) possèdent des pores, ou sont des fibres non poreuses, creuses, ou des fibres creuses sur toute leur longueur, possédant également des pores.

2. Mat composite suivant la revendication 1, caractérisé en ce que les fibres (2) se présentent sous la forme de fils ou brins (7).

3. Mat composite suivant la revendication 1, caractérisé en ce que les fibres (2) se présentent sous la forme de rovings.

4. Mat composite suivant les revendications 1 à 3, caractérisé en ce qu'une seconde feuille perméable (6) est placée par-dessus plusieurs rangées desdites fibres (2) à une extrémité du mat précité, couvrant toute la longueur des fibres précitées (2) entre les deux bandes adhésives (3, 4).

5. Cartouche à utiliser dans des systèmes de séparation de fluides, possédant un élément de boîtier supérieur (10) formant le sommet du boîtier de cartouche et un élément de boîtier inférieur (11) dans lequel les fils ou brins (7) de fibres creuses (2) et les feuilles perméables (1, 6) d'un mat composite selon les revendications 1-2 et 4 s'obtiennent par coulée et les extrémités des fibres dans l'élément de boîtier supérieur (10) sont scellées par moulage par fusion et où les bandes adhésives (3, 4) sont situées juste en-dessous de l'élément de boîtier supérieur (10) et juste au-dessus de l'élément de boîtier inférieur (11), pour servir de barrières et la feuille perméable (6) est enroulée autour d'un tube distributeur (12) centralement disposé par rapport aux fils (7) et se terminant et se logeant dans les éléments de boîtier supérieur et inférieur (10, 11), où l'extrémité du tube distributeur (12) logée dans l'élément de boîtier inférieur (11) est scellée par moulage par coulée et suivie d'une couche de fils creux (7) et ensuite de couches alternantes de feuille (1) et de fils creux (7), au fur et à mesure de l'enroulement du mat en enveloppes successives autour du tube distributeur (12).

6. Cartouche suivant la revendication 5, caractérisée en ce que les fils (7) de fibres du mat sont creux et possèdent des pores.

7. Utilisation de la cartouche suivant la revendication 5 ou 6 dans un dispositif séparateur de fluides possédant un boîtier tubulaire (20), qui comporte une conduite d'entrée de fluide (21) et une conduite de sortie de fluide (22) et un élément servant de couvercle (23) qui y est associé et assujetti de manière étanche aux parois latérales du boîtier (20), où la cartouche est logée dans le boîtier (20) et le tube distributeur est raccordé à la conduite d'entrée de fluide (21) et l'élément de boîtier inférieur (11) de la cartouche est assujetti de manière étanche aux parois latérales (20) du dispositif séparateur.

## Patentansprüche

1. Glasfaserhaltige Verbundmatte,

**gekennzeichnet durch**
eine Vielzahl Von auf einer der Hauptoberflächen einer flüssigkeitsdurchlässigen Folie (1) mit zwei Hauptoberflächen und vier Kantenoberflächen (1a, 1b, 1c, 1d) befestigten Glasfasern (2), wobei die Fasern generell parallel zueinander ausgerichtet und ihre Enden den beiden einander gegenüberliegenden Kanten der Hauptoberfläche der Folie (1) zugewandt sind, und mit mindestens zwei Klebstreifen (3, 4) parallel und in Abstand voneinander und von den Enden der Fasern, wobei die Klebstreifen (3, 4) die Fasern (2) an der Folie und aneinander fixieren und eine integrierte Verbundmatte ausbilden, in der die Fasern (2) Poren aufweisen oder nichtporöse Hohlfasern oder Hohlfasern mit Poren auf ihrer gesamten Länge sind.

2. Verbundmatte nach Anspruch 1,
   **dadurch gekennzeichnet,**
   daß die Fasern (2) in Form von Strängen (7) vorliegen.

3. Verbundmatte nach Anspruch 1,
   **dadurch gekennzeichnet,**
   daß die Fasern (2) in Form von Vorgarnen vorliegen.

4. Verbundmatte nach Ansprüchen 1 bis 3,
   **dadurch gekennzeichnet,**
   daß eine zweite durchlässige Folie (6) über zahlreichen Reihen der Fasern (2) an einem Ende der Matte angeordnet ist, die die gesamte Länge der Fasern (2) zwischen den beiden Klebstreifen (3, 4) abdeckt.

5. Patrone zum Gebrauch in Flüssigkeitstrennsystemen mit einem oberen Gehäuseteil (10), das den Kopf des Patronengehäuses bildet, und einem unteren Gehäuseteil (11), in das die Stränge (7) von Hohlfasern und die durchlässigen Folien (1, 6) einer Verbundmatte nach Ansprüchen 1 bis 2 und 4 eingegossen sind, und die Faserenden im oberen Gehäuseteil (10) durch Eingießen verschlossen sind, und die Klebstreifen (3, 4) gerade unterhalb dem oberen Gehäuseteil (10) und gerade über dem unteren Gehäuseteil (11) als Sperren angeordnet sind und die durchlässige Folie (6) um ein Verteilerrohr (12) gewickelt ist, das im Zentrum der Stränge (7), die in den oberen und unteren Gehäuseteilen (10, 11) enden und darin eingebaut sind, angeordnet ist und wobei das im unteren Gehäuseteil (11) eingebaute Ende des Verteilerrohres (12) durch Eingießen verschlossen ist, gefolgt von einer Schicht hohler Stränge (7) und dann Schichten von Folie (1) und hohlen Strängen (7) sich abwechseln, wenn die Matte in aufeinanderfolgenden Windungen um das Verteilerrohr (12) gewickelt ist.

6. Patrone nach Anspruch 5,
   **dadurch gekennzeichnet,**
   daß die Faserstränge (7) der Matte hohl sind und Poren aufweisen.

7. Verwendung der Matte nach Ansprüchen 5 oder 6 in einer Flüssigkeitstrenneinrichtung mit einem rohrförmigen Gehäuse (20) mit einem Flüssigkeitseinlaß (21) und einem Flüssigkeitsauslaß (22) und einem damit verbundenen Deckel (23), der mit den Seitenwänden des Gehäuses (20) versiegelt ist, wobei die Patrone im Gehäuse (20) angeordnet ist und das Verteilerrohr (12) mit dem Flüssigkeitseinlaß (21) verbunden ist, und das untere Gehäuseteil (11) der Patrone mit den Seitenwänden (20) der Trenneinrichtung versiegelt ist.

FIGURE 1

FIGURE 2

FIGURE 3

FIGURE 4